# EUROPÄISCHE PATENTSCHRIFT

(11) **EP 3 668 553 B1**
(45) Veröffentlichungstag und Bekanntmachung des Hinweises auf die Patenterteilung: **01.12.2021**
(21) Anmeldenummer: 18753338.5
(22) Anmeldetag: 01.08.2018
(51) Int. Cl.: A61L 2/20

(54) **DEKONTAMINATIONSVORRICHTUNG, ISOLATORSYSTEM SOWIE BETRIEBSVERFAHREN**
DECONTAMINATION DEVICE, ISOLATOR SYSTEM, AND OPERATING METHOD
DISPOSITIF DE DÉCONTAMINATION, SYSTÈME ISOLATEUR ET PROCÉDÉ DE FONCTIONNEMENT

(30) Priorität: 14.08.2017 DE 102017118481
(43) Veröffentlichungstag der Anmeldung: 24.06.2020
(73) Patentinhaber: Metall + Plastic GmbH, 78315 Radolfzell (DE)
(72) Erfinder: KLEINMANN, Stefan, 78315 Radolfzell (DE); KASSNER, Thomas, 78315 Radolfzell (DE)
(74) Vertreter: Patent- und Rechtsanwälte Behrmann Wagner PartG mbB
(86) Internationale Anmeldenummer: PCT/EP2018/070831
(87) Internationale Veröffentlichungsnummer: WO 2019/034424

(56) Entgegenhaltungen:
- EP-A1- 1 461 812
- EP-A2- 1 787 731
- WO-A1-2009/030599
- WO-A1-2011/085735
- US-A1- 2008 003 135

## Beschreibung

Die Erfindung betrifft eine Dekontaminationsvorrichtung zum Dekontaminieren von Produkten in Form von Verbrauchsgütern und/oder Verbrauchsgüterumverpackungen mit darin befindlichen, vorsterilisierten, d.h. steril verpackten, besonders bevorzugt als Primärverpackungen ausgebildeten, vorsterilisieren Verbrauchsgütern vor dem Zuführen der Verbrauchsgüter in einen Isolator, insbesondere für pharmatechnische Anwendungen.

Ferner betrifft die Erfindung ein Isolatorsystem, mit einem Isolator und dem Isolator vorgeordnete Dekontaminationsvorrichtung.

Darüber hinaus betrifft die Erfindung ein Verfahren gemäß dem Oberbegriff des Anspruchs 8 zum Betreiben einer erfindungsgemäßen Dekontaminationsvorrichtung und/oder eines erfindungsgemäßen Isolatorsystems.

Es ist bekannt, Verbrauchsgüter, wie Arzneimittelverpackungen bzw. - behälter und/oder deren Umverpackungen vor der Zuführung der Verbrauchsgüter in einen Isolator zu dekontaminieren, wozu die Verbrauchsgüter bzw. Verbrauchsgüterumverpackungen in einer Dekontaminationskammer mit Dekontaminationsmittel, meist in Form von Wasserstoffperoxyddampf beaufschlagt und danach steril dem Isolator zugeführt werden. Für den Fall der Dekontamination von Umverpackungen werden die Verbrauchsgüter zum Isolator aus der innen sterilen und wie zuvor beschriebenen dekontaminierten Umverpackung ausgepackt. Bei bekannten Dekontaminationsvorrichtungen wird als nachteilig empfunden, dass die Zeitspanne zwischen Beschickung der Dekontaminationskammer und dem Überführen in den Isolator lang ist. Dies ist insbesondere darauf zurückzuführen, dass die Dekontaminationskammer zur Vermeidung einer Belastung der Bedienperson zum Beladezeitpunkt frei ist von Dekontaminationsmittel und nach dem Schließen der Dekontaminationskammer zunächst die Dekontaminationsmittelkonzentration in der Dekontaminationskammer (wieder) angehoben, während der Dekontaminationszeit aufrechterhalten und danach vor dem Öffnen einer Verbindung zum Isolator wieder reduziert werden muss, um eine Leckage von Dekontaminationsmittel in den Isolator hinein zu vermeiden, da das Dekontaminationsmittel, meist Wasserstoffperoxyd, negative Einflüsse auf die in dem Isolator befindlichen medizinischen Wirkstoffe haben kann.

Aus der EP 1 787 731 A2 ist eine Waschvorrichtung, insbesondere zur Wärmedesinfektion, bekannt, die eine Vielzahl von Waschmaschinen umfasst, die entlang einer Ausrichtungsachse ausgerichtet angeordnet sind, und eine Bewegungsvorrichtung zum Bewegen eines objekttragenden Behälters parallel zur Ausrichtungsachse. Die Bewegungsvorrichtung ist mit einem Wagen zum Transportieren des Behälters und mit Gleitmitteln versehen, die von einem Führungselement geführt werden, das sich parallel zur Ausrichtungsachse erstreckt.

Die US 2008/003135 A1 lehrt weiterhin ein Produktsterilisations- oder Pasteurisationsverfahren. Die beschriebne Methode beinhaltet die bachfolgenden Verfahrensschritte: Übertragen eines Produkts von außen nach innen in einen druckbeaufschlagten Einlassraum einer Kammer durch mindestens eine Druckluftschleuse; Laden des Produkts auf einen Förderer, um das Produkt durch die Kammer von dem Einlassraum in einen Auslassraum zu übertragen; Erwärmen des Produkts durch Eintauchen des Produkts in hydrostatische Säulen unter Anwendung von variablem Druck und Temperatur; Durchleiten des Produkts durch einen Zwischendruckraum; Kühlen des Produkts durch Eintauchen des Produkts in hydrostatische Säulen unter Anwendung von variablem Druck und Temperatur; Entladen des Produkts vom Förderer in einem druckbeaufschlagten Auslassraum; und Übertragen des Produkts nach außen aus dem Auslassraum durch mindestens eine atmosphärische Druckluftschleuse.

Ausgehend von dem vorgenannten Stand der Technik liegt der Erfindung daher die Aufgabe zugrunde, eine verbesserte Dekontaminationsvorrichtung, ein verbessertes Isolatorsystem sowie ein entsprechendes, verbessertes Betriebsverfahren anzugeben, mit denen eine schnellere, bevorzugt quasi-kontinuierliche Beschickung des Isolators mit Verbrauchsgütern möglich ist. Insbesondere soll die Aufenthaltszeit der Produkte (insbesondere der Verbrauchsgüterumverpackung) in einer Dekontaminationskammer der Dekontaminationsvorrichtung verglichen mit dem Stand der Technik reduziert werden.

Diese Aufgabe wird hinsichtlich der Dekontaminationsvorrichtung mit den Merkmalen des Anspruchs 1 gelöst, also mit einer Dekontaminationsvorrichtung zum dekontaminieren von Produkten, insbesondere Verbrauchsgütern und/oder (bevorzugt Verbrauchsgüter, wie sterile Pharmazeutikaprimärverpackungen bzw. -behältnisse beinhaltenden, verschlossenen) Verbrauchsgüterumverpackungen vor der Zuführung der Verbrauchsgüter in einen Isolator, insbesondere eingerichtet für pharmatechnische Anwendungen, mit mehreren, jeweils eine verschließbare Eingangsöffnung sowie eine verschließbare Ausgangsöffnung aufweisenden Dekontaminationskammern, jeweils umfassend Beaufschlagungsmittel zum Beaufschlagen der Dekontaminationskammern mit Dekontaminationsmittel, insbesondere Dekontaminationsmitteldampf oder -aerosol, ganz besondere bevorzugt mit einem Wasserstoffperoxyddampf oder -aerosol, sowie mit einem den Dekontaminationskammern zugeordneten, eine Zuführöffnung für zu dekontaminierende Produkte aufweisenden Verteilerraum, umfassend Verteilermittel zum (automatisierten) Verteilen der Produkte auf die unterschiedlichen, insbesondere freien, Dekontaminationskammern und mit einem den Dekontaminationskammern nachgeordneten, eine Ausschleuseöffnung, insbesondere für dekontaminierte Produkte oder daraus entpackte Verbrauchsgüter, aufweisenden, Ausschleuseraum, umfassend Transportmittel zum Transportieren der dekontaminierten Produkte aus den Dekontaminationskammer, bevorzugt in Richtung zu der Ausschleuseöffnung und/oder zu Entpackungsmitteln, wobei dem Verteilerraum und/oder dem Ausschleuseraum Mittel zum Absenken einer Dekontaminationsmittelkonzentration in dem Verteilerraum und/oder dem Ausschleuseraum zugeordnet sind, mit denen das Dekontamiationsmittel in einem Umluftbetrieb durch einen Katalysator gefördert und/oder Dekontaminationsmittel durch Zuführen von Frischluft verdrängt wird.

Hinsichtlich des Isolatorsystems wird die Aufgabe mit den Merkmalen des Anspruch 7 gelöst.

Hinsichtlich des Verfahrens wird die Aufgabe mit den Merkmalen des Anspruchs 8 gelöst, also bei einem gattungsgemäßen Verfahren dadurch, dass zu dekontaminierende Produkte in Form von Verbrauchsgütern oder Verbrauchsgüterumverpackungen mit darin befindlichen, sterilen Verbrauchsgütern, insbesondere Primärverpackungen, insbesondere nacheinander, durch die Zuführöffnung in die Verteilerkammer eingebracht, dort mit den Verteilermitteln auf die unterschiedlichen Dekontaminationskammern verteilt und nach einer vorgegebenen Dekontaminationszeit, während derer die Dekontaminationskammern verschlossen sind, mit den Transportmitteln innerhalb des Ausschleuseraums in Richtung zu der Ausschleuseöffnung transportiert und über diese einem Isolator zugeführt werden, und dass die Dekontaminationsmittelkonzentration in den Verteilerraum und/oder dem Ausschleuseraum, bevorzugt kontinuierlich, abgesenkt bzw. reduziert wird.

Vorteilhafte Weiterbildungen der Erfindung sind in den Unteransprüchen angegeben.

In den Rahmen der Erfindung fallen sämtliche Kombinationen aus zumindest zwei von in der Beschreibung, den Ansprüchen und/oder den Figuren offenbarten Merkmalen.

Zur Vermeidung von Wiederholungen sollen vorrichtungsgemäß offenbarte Merkmale auch als verfahrensgemäß offenbart gelten und beanspruchbar sein. Ebenso sollen verfahrensgemäß offenbarte Merkmale auch als vorrichtungsgemäß offenbart gelten und beanspruchbar sein.

Der Erfindung liegt zunächst der Gedanke zugrunde, mehrere, bevorzugt mehr als zwei, Dekontaminationskammern vorzusehen, die insbesondere nacheinander mit zu dekontaminierenden Produkten in Form von Verbrauchsgütern und/oder Verbrauchsgüterumverpackungen mit darin angeordneten sterilen Verbrauchsgütern, beladbar sind, und zwar über in einem Verteilerraum angeordnete Verteilermittel, über die die Produkte automatisiert auf die unterschiedlichen Dekontaminationskammern verteilbar sind. Die Dekontaminationskammern weisen jeweils sowohl eine Eingangs- als auch eine Ausgangsöffnung auf, wobei sowohl die Eingangsöffnung als auch die Ausgangsöffnung verschließbar sind, wobei die jeweilige Eingangsöffnung zum Beladen geöffnet und danach wieder geschlossen und die Ausgangsöffnung zum Entladen geöffnet und danach wieder verschlossen wird. Die Ausgangsöffnungen münden in einen den Dekontaminationskammern nachgeordneten Ausschleuseraum, in dem die fertig dekontaminierten Produkte mithilfe von Transportmitteln von bzw. aus den einzelnen Dekontaminationskammern abgeholt und, bevorzugt in Richtung der Ausschleuseöffnung, transportiert werden bzw. transportierbar sind, über die eine Zuführung (unmittelbar oder mittelbar über eine fakultative Produktschleuse) hin zum Isolator möglich ist. Ganz besonders bevorzugt handelt es sich bei den zu dekontaminierenden Produkten um Verbrauchsgüterumverpackungen, die über die Ausschleuseöffnung einer als Entpackungsschleuse ausgebildeten Produktschleuse zugeführt werden, innerhalb derer aus den dekontaminierten Produkten, d.h. den Verbrauchsgüterumverpackungen vorsterilisierte, d.h. sterilisierte Verbrauchsgüter, insbesondere Primärverpackungen für Pharmazeutika, entweder manuell oder automatisiert entpackt werden und dann diese sterilen, entpackten Verbrauchsgüter aus der Produktschleuse steril in den Isolator überführt werden. Grundsätzlich denkbar ist auch eine alternative Nutzung der erfindungsgemäßen Dekontaminationsvorrichtung, bei der nicht die Verbrauchsgüterumverpackungen, sondern die dann die Produkte bildenden Verbrauchsgüter unmittelbar dekontaminiert und über den Ausschleuseraum, ggf. unter der Zwischenschaltung einer Produktschleuse dem Isolator zugeführt werden.

Der Verteilerraum und der Ausschleuseraum haben erfindungsgemäß jedoch nicht nur eine Verteil- bzw. Einsammelfunktion bezogen auf die Produkte, sondern zumindest einem dieser beiden Räume, insbesondere beiden Räumen, sind Mittel zur Reduzierung der Dekontaminationsmittelkonzentration in der (jeweiligen) Raumluft zugeordnet, um die Konzentration von Dekontaminationsmittel im Verteilerraum und/oder dem Ausschleuseraum zu reduzieren. Diese Ausführungsform ermöglicht eine Ausbildung und Betriebsweise der Dekontaminationsvorrichtung, bei welcher die Dekontaminationsmittelkonzentration in den Dekontaminationskammern während des Betriebs der Dekontaminationsvorrichtung dauerhaft auf einem hohen Niveau gehalten werden kann, d.h. zum Beschicken der Dekontaminationskammern und zum Entleeren der Dekontaminationskammern muss die Dekontaminationsmittelkonzentration in den Dekontaminationskammern nicht wie im Stand der Technik zuvor auf gegen null reduziert werden, sondern die Dekontaminationskammern können bei hoher Dekontaminationsmittelkonzentration beschickt und entladen werden, wodurch die im Stand der Technik notwendigen Rampen zum Aufbau und zum Abbau der Dekontaminationsmittelkonzentration vermieden werden können und dadurch die Aufenthaltszeit der Produkte in der jeweiligen Dekontaminationskammer reduziert werden kann. Das während der Beladung in den Verteilerraum über die Eingangsöffnungen der Dekontaminationskammern einströmende Dekontaminationsmittel und/oder das während der Entladung über die Ausgangsöffnungen in den Ausschleuseraum einsströmende Dekontaminationsmittel bzw. die hieraus resultierende Dekontaminationsmittelkonzentration im Verteilerraum und/oder im Auschleuseraum kann durch die Absenkmittel reduziert werden, erfindungsgemäß indem das Dekontaminationsmittel im Umluftbetrieb durch einen Katalysator gefördert und/oder Dekontaminationsmittel durch Zuführen von Frischluft verdrängt wird.

Insgesamt ermöglicht die erfindungsgemäße Dekontaminationsvorrichtung bzw. ein damit ausgestattetes Isolatorsystem sowie das erfindungsgemäße Betriebsverfahren eine schnellere Beschickung eines Isolators bei reduzierter Aufenthaltsdauer der zu dekontaminierenden Produkte in den Dekontaminationskammern. Darüber hinaus kann durch die Nacheinanderbeschickung einer Mehrzahl von dann zeitversetzt und überlappend die Produkte dekontaminierenden Dekontaminationskammern ein quasikontinuierlicher Betrieb bzw. eine quasi-kontinuierliche Versorgung des Isolators mit Verbrauchsgütern sichergestellt werden. Dabei ist es grundsätzlich, wie erläutert, möglich unmittelbar die Verbrauchsgüter zu dekontaminieren und dann dem Isolator zuzuführen - bevorzugt ist eine Ausführungsform, bei der die dem Isolator zuzuführenden Verbrauchsgüter in eine Verbrauchsgüterumverpackung (vorsterilisiert) angeordnet sind und die Umverpackung innerhalb der Dekontaminationskammer dekontaminiert wird, woraufhin bzw. wonach die Verbrauchsgüter, entweder im Isolator oder bevorzugt vor der Zuführung zum Isolator, beispielsweise im Ausschleuseraum oder einer fakultativen, dem Ausschleuseraum nachgeordneten, dann als Entpackungsschleuse ausgebildeten Produktschleuse entpackt werden.

Für eine flächensparende Ausbildung der Dekontaminationsvorrichtung ist in Weiterbildung der Erfindung mit Vorteil vorgesehen, dass die Dekontaminationskammern entlang einer Vertikalen übereinander angeordnet sind, und dass die Verteilermittel und die Transportmittel jeweils einen Produktaufzug umfassen, mit dem die Produkte (im Falle der Verteilermittel) den einzelnen, übereinander angeordneten Dekontaminationskammern zuführbar bzw. (im Falle der Transportmittel) von diesen einzelnen, übereinander angeordneten Dekontaminationskammern hin zu der Ausschleuseöffnung des Ausschleuseraums transportierbar sind. Grundsätzlich ist als Produktaufzug eine Paternoster-Lösung realisierbar - bevorzugt ist jedoch eine Produktaufnahme bzw. Plattform umfassende klassische Aufzugslösung, bei der die Produktaufnahme ohne Umlenkung wie bei einem Paternoster entlang der Vertikalen auf derselben Verstellstrecke hin- und herverfährt.

Im Hinblick auf die konkrete Ausgestaltung der Mittel zum Absenken der Dekontaminationsmittelkonzentration im Verteilerraum und/oder im Ausschleuseraum gibt es unterschiedliche Möglichkeiten. Ganz besonders bevorzugt ist eine Ausführungsform dieser Absenkmittel, bei der diese Umlufterzeugermittel, insbesondere in Form eines Gebläses umfassen, mit denen ein Umluftvolumenstrom der jeweiligen Raumluft durch Katalysatormittel zum Abbauen des Dekontaminationsmittels erzeugbar ist. Ferner umfassen die Absenkmittel dann derartige Katalysatormittel. Die, beispielsweise paladiumhaltigen, Katalysatormittel sind dabei bevorzugt derart ausgebildet, dass sie das Dekontaminationsmittel, insbesondere Wasserstoffperoxyd in seine Bestandteile, im Falle von Wasserstoffperoxyd bevorzugt in Wasser und Sauerstoff zerlegen. Zusätzlich oder alternativ können die Absenkmittel Frischluftversorgungsmittel zum Verdrängen von dekontaminationsmittelhaltiger Raumluft aus dem jeweiligen Raum, insbesondere in die Atmosphäre, ganz besonders bevorzugt über einen Katalysator zum Abbau des Dekontaminationsmittels umfassen.

Wie erläutert, umfasst der Verteilerraum bevorzugt Mittel zum Absenken der Dekontaminationsmittelkonzentration. Da sich in der Praxis jedoch eine gewisse Dekontaminationsmittelkonzentration im Verteilerraum nicht vermeiden lässt, ist in Weiterbildung der Erfindung zur Reduzierung einer Gefahr, insbesondere einer Verätzungsgefahr, für eine Belade- bzw. Bedienperson eine Produktschleuse vorgesehen bzw. dem Verteilerraum vorgeordnet, die eine verschließbare Eingangstür aufweist und über die bei geschlossener Eingangstür mindestens ein darin befindliches Produkt über die Zuführöffnung des Verteilerraums dem Verteilerraum zuführbar ist. Die Produktschleuse umfasst Mittel zum Absenken der Dekontaminationsmittelkonzentration, beispielsweise in Form von Umlufterzeugungsmittel und Katalysatormitteln zum Abbau bzw. zur Zerlegung von Dekontaminationsmittel und/oder Frischluftversorgungsmittel zum Reduzieren der Dekontaminationsmittelkonzentration in der Schleuse vor einem Öffnen der Eingangstür. Aufgrund des verglichen mit dem Verteilerraum kleineren Innenvolumens der Produktschleuse kann die Dekontaminationsmittelkonzentration auf unbedenkliche Werte, insbesondere gegen null reduziert werden, bevor die Eingangstür geöffnet wird und/oder sich öffnen lässt für die Zufuhr neuer bzw. zu dekontaminierender Produkte.

Ganz besonders bevorzugt ist eine Ausführungsform, bei der dem Ausschleuseraum eine Produktschleuse nachgeordnet ist, die bevorzugt von einem Isolator über mindestens eine (weitere) Produktschleusentür trennbar und bevorzugt über die Auslassöffnung des Ausschleuseraums zugänglich ist. In der Produktschleuse sind bevorzugt Auspackmittel zum automatisierten Auspacken von Verbrauchsgütern aus einer im Rahmen des Dekontaminationsprozesses dekontaminierten Umverpackung angeordnet. Zusätzlich oder alternativ ist eine manuelle Entpackung, insbesondere über in einer Seitenwand angeordnete Handschuhe von außen her möglich bzw. realisierbar. Bevorzugt ist eine Dekontaminationsmittelkonzentration in der Produktschleuse vor dem Öffnen einer Schleusentür in Richtung Isolator und bevorzugt nach Verschließen in einer der Verbindung zum Ausschleuseraum durch entsprechende Mittel absenkbar. Anders ausgedrückt umfasst die Produktschleuse Mittel zum Absenken der Dekontaminationsmittelkonzentration, erfindungsgemäß in Form von Umlufterzeugungsmitteln und Katalysatormitteln zum Abbau bzw. zur Zerlegung von Dekontaminationsmittel und/oder Frischluftversorgungsmittel zum Reduzieren der Dekontaminationsmittelkonzentration in der Schleuse.

Bevorzugt umfasst die Dekontaminationsvorrichtung Mittel zum Überführen von Produkten von den Verteilermitteln in eine jeweilige Dekontaminationskammer. Dabei können diese Überführmittel Bestandteil der Verteilermittel sein, beispielsweise indem eine Produktaufnahme der Verteilermittel entsprechend angetriebene Transportrollen und/oder ein angetriebenes Transportband und/oder einen Produktschieber umfasst. Denkbar ist auch eine Anordnung dieser Überführmittel außerhalb der Verteilermittel, insbesondere in einem Bereich vor einer jeweiligen Dekontaminationskammer im Verteilerraum. Zusätzlich oder alternativ umfasst die Dekontaminationsvorrichtung bevorzugt Mittel zum Entnehmen von dekontaminierten Produkten aus den Dekontaminationskammern bzw. zum Überführen auf die Transportmittel, wobei diese in den Dekontaminationskammern und/oder an den Transportmitteln, beispielsweise in Form von Transportrollen und/oder einem Förderband und/oder einem Schieber oder einer Zugeinrichtung realisiert sein können. Zusätzlich oder alternativ umfasst die Dekontaminationsvorrichtung bevorzugt Mittel zum Fördern der Produkteausrichtung der jeweiligen Eingangsöffnung der Dekontaminationskammer zu der jeweiligen Ausgangsöffnung. Auch hier können Schieber und/oder Zugmittel und/oder angetriebene Rollen und/oder angetriebene Bänder etc. eingesetzt werden.

Wie bereits angedeutet ist die Dekontaminationsvorrichtung bevorzugt derart betreibbbar, dass die Dekontaminationsmittelkonzentration während des Betriebs der Vorrichtung, d.h. auch während einer Be- und Entladung der Dekontaminationskammern in diesen auf einem hohen Niveau bleibt. Ganz besonders bevorzugt sind hierzu die Beaufschlagungsmittel zum Beaufschlagen der Dekontaminationskammern mit Dekontaminationsmittel so angesteuert, dass eine, insbesondere überwachte bzw. gemessene Dekontaminationsmittelkonzentration in den Dekontaminationskammern auch während des Beladens und/oder Entladens der Dekontaminationskammern oberhalb eines Grenzwertes, insbesondere aus einem Wertebereich zwischen 300 ppm und 1200 ppm, ganz besonders bevorzugt 500 ppm und 1000 ppm gehalten wird bzw. haltbar ist.

Ganz besonders bevorzugt ist eine Ausführungsform der Dekontaminationsvorrichtung, bei der diese in den Dekontaminationskammern jeweils Hebemittel zum Anheben der zu dekontaminierenden Produkte umfasst, wobei die Hebemittel bevorzugt derart ausgebildet sind, dass eine entlang der Vertikalen untere Bodenseite zumindest während eines Zeitabschnitts der Dekontaminationsphase frei schwebt bzw. zur Sicherstellung eines Kontaktes mit Dekontaminationsmitteln nicht verdeckt ist.

Ferner betrifft die Erfindung ein Isolatorsystem, insbesondere für pharmatechnische Anwendungen, umfassend einen Isolator, sowie eine an diese derart angeschlossene nach dem Konzept der Erfindung ausgebildete Dekontaminationsvorrichtung, dass aus dieser dekontaminierte Produkte in Form von Verbrauchsgütern oder im Falle der Ausbildung der Produkte als Verbrauchsgüterumverpackung daraus, bevorzugt zuvor entpackte, sterile bzw. vorsterilisierte Verpackungsgüter, insbesondere Primärverpackungen aus entweder unmittelbar dem Ausschleuseraum oder über eine zusätzliche (fakultative) Produktschleuse, in dem Isolator, insbesondere in einen Manipulatorraum des Isolators steril zuführbar sind. Ganz besonders bevorzugt werden die Verbrauchsgüter in der Produktschleuse vor dem Zuführen zum Isolator aus der jeweiligen, dekontaminierenden Umverpackung (manuell oder automatisiert) entpackt. Bevorzugt umfasst der Isolator, bevorzugt ein Manipulatorraum des Isolators, Einrichtungen zur Produktion und/oder zum Abfüllen und/oder zur Bearbeitung pharmazeutischer Wirkstoffe. Die aus der erfindungsgemäßen Dekontaminationsvorrichtung in den Isolator hinein transportierten Produkte werden jedenfalls bevorzugt für pharmatechnische Anwendungen, insbesondere zur Befüllung mit einem pharmatechnischen Wirkstoff innerhalb des Isolators verwendet. Ganz besonders bevorzugt werden bereits Verbrauchsgüter, insbesondere Verpackungen aus einer in einer Dekontaminationskammer der Dekontaminationsvorrichtung dekontaminierten, die bereits sterilen Verbrauchsgüter beinhaltenden Verbrauchsgüterverpackung innerhalb des Isolators oder alternativ vor der Zuführung, entweder im Ausschleuseraum oder einer fakultativen, dieser nachgeordneten Produktschleuse entpackt und bestimmungsgemäß verwendet. Der, insbesondere für pharmatechnische Anwendungen eingerichtete Isolator umfasst einen dekontaminierbaren Raum, insbesondere den vorerwähnten Manipulatorraum und/oder einen von dem Manipulatorraum über eine Membran zur Laminarisierung eines Luftstroms, getrennten Plenumraum. Ein Umluftgebläse zur Ergänzung des Luftstroms ist bevorzugt in einem Umlufterzeugerraum oberhalb des vorerwähnten Plenumraumes angeordnet. Ganz besonders bevorzugt ist der Manipulatorraum über einen, weiter bevorzugt zwischen zwei transparenten Scheiben oder alternativ von einer Rohrleitung ausgebildeten, Ruckströmkanal luftleitend mit dem vorerwähnten, bevorzugt vorgesehenen Umlufterzeugerraum verbunden. Bevorzugt ist zur Realisierung des Merkmals der Einrichtung des Isolators für pharmazeutische bzw. pharmatechnische Anwendungen in dem Manipulatorraum eine Dosier- und/oder eine Abfülleinrichtung für einen pharmazeutischen Wirkstoff und/oder eine Herstellungseinrichtung für einen solchen Wirkstoff angeordnet. Ganz besonders bevorzugt ist es, wenn der Manipulatorraum über Handschuhe für einen manuellen Eingriff von außen für eine Bedienperson zugänglich ist, wobei die Handschuhe bzw. sogenannte Handschuhports in einer Seitenwand des Manipulators angeordnet bzw. festgelegt sind. Bevorzugt ist zwischen dem Ausschleuseraum und dem Isolator eine, bevorzugt als Entpackungsschleuse ausgebildete Produktschleuse angeordnet, in der Verbrauchsgüter, insbesondere Primärverpackungen aus einer zuvor in einer der Dekontaminationskammern dekontaminierten Umverpackung, bevorzugt automatisiert oder alternativ manuell entpackbar sind. Der Produktschleuse sind dabei Mittel zum Absenken der Dekontaminationsmittelkonzentration vorgesehen, insbesondere nach einer Trennung vom Ausschleuseraum und vor dem Öffnen einer Verbindung zum Isolator.

Ferner führt die Erfindung auf ein Verfahren zum Betreiben einer erfindungsgemäßen Dekontaminationsvorrichtung und/oder eines erfindungsgemäßen Isolatorsystems. Im Rahmen des Verfahrens werden zu dekontaminierende Produkte in Form von Verbrauchsgütern wie Produktverpackungen und/oder die Verbrauchsgüter bereits steril beinhaltenden Verbrauchsgüterumverpackungen, insbesondere nacheinander, durch die Zuführöffnung, in den Verteilerraum eingebracht und dort mit den Verteilermitteln, insbesondere entlang einer Vertikalen, auf die bevorzugt entlang der Vertikalen übereinander angeordneten, Dekontaminationskammern verteilt und nach einer vorgegebenen Dekontaminationszeit, während derer die Dekontaminationskammern, d.h. deren jeweilige Eingangs- und/oder Ausgangsöffnung verschlossen sind mit den Transportmitteln innerhalb des Ausschleuseraums, insbesondere entlang einer Vertikalen zu der Ausschleuseöffnung transportiert und über diese einem Isolator zugeführt werden, wobei, insbesondere kontinuierlich und/oder während eines Belade- und/oder Entladevorgangs der Dekontaminationskammern und/oder während einer Dekontaminationsphase, bei der zumindest einer der Dekontaminationskammern geschlossen ist, die Dekontaminationsmittelkonzentration in den Verteilerraum und/oder dem Ausschleuseraum, ganz besonders bevorzugt durch Fördern der jeweiligen Raumluft im Umluftbetrieb durch einen Katalysator bzw. Katalysatormittel, die eingerichtet sind zum Abbau von Dekontaminationsmittel gefördert wird.

Besonders bevorzugt ist es, wenn die Dekontaminationsmittelkonzentration während des normalen Betriebs der Dekontaminationsvorrichtung, d.h. insbesondere während des Be- und Entladens der Dekontaminationskammern aus dem Verteilerraum bzw. in den Ausschleuseraum oberhalb eines Grenzwertes, insbesondere zwischen 300 ppm und 1200 ppm gehalten wird, um somit im Stand der Technik notwendige Konzentrationserhöhungs- und/oder Konzentrationsabsenkungsrampen zu vermeiden. Zusätzlich oder alternativ wird auf ein aktives Absenken der Dekontaminationsmittelkonzentration durch den Einsatz entsprechender Absenkmittel, wie Frischluftzuführungsmittel und/oder Umlufterzeugungsmittel, mit denen das Dekontaminationsmittel bzw. die Raumluft der Dekontaminationskammern im Umluftbetrieb durch einen Katalysator zum Abbau des Dekontaminationsmittels gefördert wird vor dem Be- und/oder Entladen der Dekontaminationskammer verzichtet. Die einzige (eher geringfügige) Absenkung der Dekontaminationsmittelkonzentration rührt aus der kurzen Öffnung der Verbindung der jeweiligen Dekontaminationskammer zum Verteilerraum und/oder zum Ausschleuseraum.

Weitere Vorteile, Merkmale und Einzelheiten der Erfindung ergeben sich aus der nachfolgenden Beschreibung eines bevorzugten Ausführungsbeispiels sowie anhand der Zeichnungen.

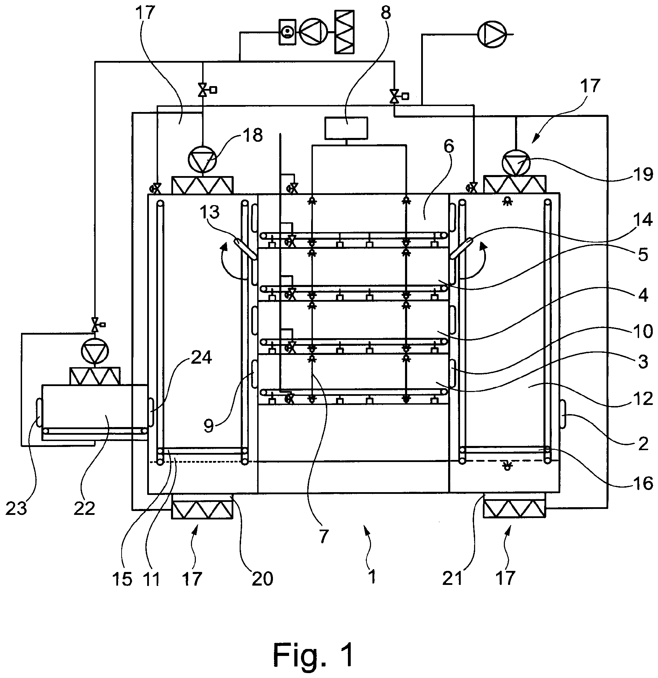

Diese zeigen in der einzigen
- Fig. 1: einen schematischen Aufbau einer bevorzugten Ausführungsform einer nach dem Konzept der Erfindung ausgebildeten Dekontaminationsvorrichtung.

In Fig. 1 ist eine nach dem Konzept der Erfindung ausgebildete Dekontaminationsvorrichtung 1 gezeigt. Diese ist einem nicht gezeigten, pharmatechnischen Isolator vorgeordnet, der über eine Ausschleuseöffnung 2 mit dekontaminierten Produkten versorgbar ist.

Die Dekontaminationsvorrichtung 1 umfasst mehrere entlang einer Vertikalen übereinander angeordnete Dekontaminationskammern 3, 4, 5, 6, denen Beaufschlagungsmittel 7 zur jeweiligen Beaufschlagung mit Dekontaminationsmittel zugeordnet sind. Im vorliegenden Fall umfassen die Beaufschlagungsmittel 7 einen Blitzverdampfer 8 zum Erzeugen eines Dekontaminationsmitteldampfes, vorliegend eines Wasserstoffperoxyddampfes sowie in den Dekontaminationskammern 3, 4, 5, 6 ausmündende Verteilerleitungen. Zusätzlich oder alternativ können die Beaufschlagungsmittel 7 Verneblungsdüsen zum Erzeugen eines Dekontaminationsmittelaerosols, insbesondere eines Wasserstoffperoxydaerosols umfassen.

Jede Dekontaminationskammer 3, 4, 5, 6 weist eine öffen- und verschließbare Eingangsöffnung 9 sowie eine öffen- und verschließbare Ausgangsöffnung 10 auf. Über die geöffneten Eingangsöffnungen 9 können die Dekontaminationskammern 3, 4, 5, 6 mit zu dekontaminierenden Produkten aus einem Verteilerraum 11 versorgt werden, während über die geöffneten Ausgangsöffnungen 10 dekontaminierte Produkte in einen die Ausschleusöffnung 2 aufweisenden Ausschleuseraum 12 überführt werden können. Während einer Dekontaminationsphase, in der sich innerhalb einer jeweiligen Dekontaminationskammer 3, 4, 5, 6 mindestens ein zu dekontaminierendes Produkt befindet sind die Eingangsöffnung 9 und die Ausgangsöffnung 10 der jeweiligen Dekontaminationskammer 3, 4, 5, 6, verschlossen. Im vorliegenden Fall umfassen die Eingangsöffnungen 9 und die Ausgangsöffnungen 10 zum Öffnen und Verschließen entsprechende, hier beispielhaft klappenartige Türen 13, 14. Alternative Verschlusselemente (Türen) sind realisierbar.

Im Verteilerraum 11 befinden sich Verteilermittel 15 in Form eines entlang der Vertikalen verfahrbaren Produktaufzugs, über den die einzelnen Eingangsöffnungen 9 bzw. Dekontaminationskammern 3, 4, 5, 6 zum Verteilen der zu dekontaminierenden Produkte angefahren werden können. Im Ausschleuseraum 12 sind Transportmittel 16, ebenfalls in Form eines Produktaufzugs vorgesehen, um die dekontaminierten Produkte von den Dekontaminationskammern 3, 4, 5, 6 in Richtung der Ausschleuseöffnung 2 fördern bzw. transportieren zu können.

Sowohl dem Verteilerraum 11 als auch dem Ausschleuseraum 12 sind Mittel 17 zum Absenken der Dekontaminationsmittelkonzentration zugeordnet, jeweils umfassend ein Umluftgebläse 18, 19 zum Erzeugen eines Umluftvolumenstroms durch Katalysatormittel 20, 21 zum Abbauen von Dekontaminationsmitteln, hier zum Aufspalten von Wasserstoffperoxyd in die Bestandteile Wasser und Sauerstoff. Ohne diese Mittel 17 würde sich die Dekontaminationsmittelkonzentration im Verteilerraum 11 sowie im Ausschleuseraum 12 durch die zeitweise geöffneten Eingangsöffnungen 9 und Ausgangsöffnungen 10 der Dekontaminationsmittelkonzentration in den Dekontaminationskammern 3, 4, 5, 6 annähern, was vermieden werden soll. Während des Betriebs wird die Dekontaminationsmittelkonzentration in den Dekontaminationskammern 3, 4, 5, 6 weitgehend aufrechterhalten und unterschreitet bevorzugt einen vorgegebenen Grenzwert nicht. Auf ein Absenken der Dekontaminationsmittelkonzentration, insbesondere der Wasserstoffperoxydkonzentration vor dem Be- und/oder Entladen der Dekontaminationskammern 3, 4, 5, 6, d.h. vor dem Öffnen der (Eingangs-) Tür 13 und der (Ausgangs-)Tür 14 wird bewusst verzichtet. Dies wird dadurch unterstützt, dass die Öffnungszeiten der Eingangsöffnungen 9 und Ausgangsöffnungen 10 knapp bemessen ist und diese die meiste Zeit während des Betriebs geschlossen bleiben. Die Dekontaminationsmittelkonzentration in den Dekontaminationskammern 3, 4, 5, 6 wird bevorzugt gemessen und entsprechend Dekontaminationsmittel nachgeführt. Auch ist es denkbar, dass in regelmäßigen Zeitabständen, ohne eine Messung, basierend auf Erfahrungswerten oder Berechnungen Dekontaminationsmittel nachgeführt bzw. nachversorgt wird.

Wie sich aus Fig. 1 ergibt sind sowohl dem Verteilerraum 11 als auch dem Ausschleuseraum 12 Luftfilter, bevorzugt Hochleistungsschwebstofffilter zugeordnet, durch die die Luft im Umluftbetrieb zusätzlich zu den Katalysatormitteln 20, 21 gefördert wird.

Vorliegend wird der Verteilerraum 11 über eine fakultative, zusätzlich vorgesehene bzw. dem Verteilerraum 11 vorgeordnete Produktschleuse 22 mit Eingangstür 23 mit zu dekontaminierenden Produkten versorgt, wobei vor dem Öffnen der Eingangstür 23 die Dekontaminationsmittelkonzentration in der Produktschleuse 22 auf ein für eine Bedienperson ungefährliches Maß reduziert wird, vorliegend durch Fördern von Raumluft durch einen entsprechenden Katalysator im Umluftbetrieb. Aus der Produktschleuse 22 gelangen die zu dekontaminierenden Produkte über eine verschließbare Zuführöffnung 24 in den Verteilerraum 12 zu den Verteilermitteln 15.

Zu erkennen ist, dass auf den Verteilermitteln 15 sowie den Transportmitteln 16 als auch in den jeweiligen Dekontaminationskammern 3, 4, 5, 6 Fördermittel in Form von rotierenden Endlosbändern vorgesehen sind. Alternative Förder- oder Überführmittel sind realisierbar. Die Fördermittel dienen bei den Verteilermitteln dazu, die zu dekontaminierenden Produkte in die jeweilige Dekontaminationskammer 3, 4, 5, 6 zu fördern. In den Dekontaminationskammern 3, 4, 5, 6 dienen die Fördermittel dazu die Produkte von der jeweiligen Eingangsöffnung 9 zu der jeweiligen Ausgangsöffnung 10 zu transportieren. Die Fördermittel der Transportmittel 16 dienen dazu die Produkte zur bzw. durch die Ausschleuseöffnung 2 zu transportieren. Auch sind Fördermittel in der Produktschleuse 22 vorgesehen, um den Verteilerraum 11 bei geschlossener Eingangstür 23 automatisch mit zu dekontaminierenden Produkten, insbesondere Verbrauchsgüterumverpackungen mit derartig steril verpackten Verbrauchgütern, insbesondere Primärverpackungen beschicken zu können.

Bevorzugt ist dem Ausschleuseraum 12, insbesondere der Ausschleuseöffnung 2 des Ausschleuseraums 12 eine (nicht gezeigte) Produktschleuse nachgeordnet, über die die Verbrauchsgüter dem Isolator zuführbar sind und/oder in der, insbesondere automatisiert über eine entsprechende Entpackungsrobotik bzw. -maschinerie aus der zuvor dekontaminierten Verbrauchsgüterumverpackung entpackbar sind. Die Produktschleuse ist von dem Isolator über mindestens eine Schleusentür getrennt, wobei der Produktschleuse bevorzugt Mittel zum Abbau von Dekontaminationsmittel zugeordnet sind, insbesondere analog zu der Produktschleuse durch Fördern des Produktschleuseninnenraumvolumens bzw. Gases im Umluftbetrieb durch einen Katalysator und/oder durch Verdrängen von Dekontaminationsmittel mittels steriler Frischluft. Bevorzugt wird das Dekontaminationsmittel in der Produktschleuse abgebaut, bevor die Produktschleusentür zum Isolator öffnet und/oder nachdem die Verbindung zwischen dem Ausschleuseraum und der Produktschleuse geschlossen wurde.

Bei einer alternativen Ausführungsform ist es möglicht, die Verbrauchsgüter aus den Verbrauchsgüterumverpackungen bereits im Ausschleuseraum zu entpacken, bevorzugt, jedoch nicht zwingend dann unter Verzicht auf eine dem Ausschleuseraum nachgeordnete Produktschleuse. Ebenfalls ist es denkbar, mit oder ohne Realisierung der einen zwischen Ausschleuseraum und Isolator angeordneten Produktschleuse im Falle der Ausbildung der Produkt als Verbrauchsgüterumverpackungen die Verbrauchsgüter erst im Isolator aus den Produkten, sprich den Verbrauchsgüterumverpackungen zu entpacken.

### Bezugszeichen

- 1: Dekontaminationsvorrichtung
- 2: Ausschleuseöffnung des Ausschleuseraums
- 3: Dekontaminationskammer
- 4: Dekontaminationskammer
- 5: Dekontaminationskammer
- 6: Dekontaminationskammer
- 7: Beaufschlagungsmittel
- 8: Blitzverdampfer
- 9: Eingangsöffnung
- 10: Ausgangsöffnung
- 11: Verteilerraum
- 12: Ausschleuseraum
- 13: Tür
- 14: Tür
- 15: Verteilermittel
- 16: Transportmittel
- 17: Mittel zum Absenken der Dekontaminationsmittelkonzentration
- 18: Umluftgebläse
- 19: Umluftgebläse
- 20: Katalysatormittel
- 21: Katalysatormittel
- 22: Produktschleuse
- 23: Eingangstür der Produktschleuse
- 24: Zuführöffnung des Verteilerraums

## Patentansprüche

1. Dekontaminationsvorrichtung (1) zum Dekontaminieren von Produkten in Form von Verbrauchsgütern und/oder Verbrauchsgüterumverpackungen mit darin befindlichen Verbrauchsgütern vor dem Zuführen der Verbrauchsgüter in einen Isolator, mit mehreren, jeweils eine verschließbare Eingangsöffnung (9) sowie eine verschließbare Ausgangsöffnung (10) aufweisenden Dekontaminationskammern (3, 4, 5, 6), jeweils umfassend Beaufschlagungsmittel (7) zum Beaufschlagen der Dekontaminationskammern (3, 4, 5, 6) mit einem Dekontaminationsmittel (7), sowie mit einem den Dekontaminationskammern (3, 4, 5, 6) vorgeordneten, eine Zuführöffnung (24) für zu dekontaminierende Produkte aufweisenden Verteilerraum (11), umfassend Verteilermitteln (15) zum Verteilen der Produkte auf die unterschiedlichen Dekontaminationskammern (3, 4, 5, 6) und mit einem den Dekontaminationskammern (3, 4, 5, 6) nachgeordneten, eine Ausschleuseöffnung (2) aufweisenden Ausschleuseraum (12), umfassend Transportmittel (16) zum Transportieren der dekontaminierten Produkte aus den Dekontaminationskammern (3, 4, 5, 6), wobei dem Verteilerraum (11) und/oder dem Ausschleuseraum (12) Mittel (17) zum Absenken einer Dekontaminationsmittelkonzentration zugeordnet sind, mit denen das Dekontamiationsmittel in einem Umluftbetrieb durch einen Katalysator gefördert und/oder Dekontaminationsmittel durch Zuführen von Frischluft verdrängt wird.

2. Dekontaminationsvorrichtung nach Anspruch 1,
**dadurch gekennzeichnet,**
**dass** die Dekontaminationskammern (3, 4, 5, 6) entlang einer Vertikalen übereinander angeordnet sind und dass die Verteilermittel (15) und die Transportmittel (16) jeweils einen Produktaufzug umfassen, mit dem die Produkte den einzelnen, übereinander angeordneten Dekontaminationskammern (3, 4, 5, 6) zuführbar bzw. von diesen zu der Ausschleuseöffnung (2) transportierbar sind.

3. Dekontaminationsvorrichtung nach einem der vorhergehenden Ansprüche,
**dadurch gekennzeichnet,**
**dass** die Mittel (17) zum Absenken der Dekontaminationsmittelkonzentration Umlufterzeugungsmittel zum Erzeugen eines Umluftvolumenstroms durch Katalysatormittel (20, 21) zum Abbauen des Dekontaminationsmittel und/oder Frischluftversorgungsmittel zum Freispülen eines Raumvolumens mit, insbesondere steriler, Frischluft umfassen.

4. Dekontaminationsvorrichtung nach einem der vorhergehenden Ansprüche,
**dadurch gekennzeichnet,**
**dass** dem Verteilerraum (11) eine Eingangstür (23) aufweisende Produktschleuse vorgeordnet ist, die mit zu dekontaminierenden Produkten beladbar ist, die Produkte aus der Produktschleuse (22) bei geschlossener Eingangstür (23) über die Zuführöffnung (24) dem Verteilerraum (11) zuführbar sind und/oder dass dem Ausschleuseraum (12) eine Produktschleuse nachgeordnet ist, über die die Verbrauchsgüter einem Isolator zuführbar sind und/oder in der Verbrauchsgüter, manuell oder automatisiert über Entpackungsmittel vor der Zuführung zum Isolator aus den dekontaminierten, als Verbrauchsgüterumverpackung ausgebildeten Produkten entpackbar sind.

5. Dekontaminationsvorrichtung nach einem der vorhergehenden Ansprüche,
**dadurch gekennzeichnet,**
**dass** an den Verteilermitteln (15) Mittel zum Überführen von Produkten in die Dekontaminationskammern (3, 4, 5, 6) und/oder an den Transportmitteln (16), Mittel zum Entnehmen von dekontaminierten Produkten aus den Dekontaminationskammern (3, 4, 5, 6) und/oder,in den Dekontaminationskammern (3, 4, 5, 6), Mittel zum Fördern der Produkte aus Richtung der jeweiligen Eingangsöffnung (9) zu der jeweiligen Ausgangsöffnung (10) vorgesehen sind.

6. Dekontaminationsvorrichtung nach einem der vorhergehenden Ansprüche,
**dadurch gekennzeichnet,**
**dass** in den Dekontaminationskammern Hebemittel zum Anheben der Produkte angeordnet sind.

7. Isolatorsystem für pharmatechnische Anwendungen, umfassend einen Isolator sowie eine an diesen derart angeschlossene Dekontaminationsvorrichtung (1) nach einem der vorhergehenden Ansprüche, dass aus dieser dekontaminierte Produkte oder daraus zuvorin einer Produktschleuse entpackte Verbrauchsgüter dem Isolator steril zuführbar sind.

8. Verfahren zum Betreiben einer Dekontaminationsvorrichtung (1) nach einem der Ansprüche 1 bis 6 und/oder eines Isolatorsystems nach Anspruch 7, **dadurch gekennzeichnet,**
**dass** zu dekontaminierende Produkte, insbesondere nacheinander, durch die Zuführöffnung in den Verteilerraum (11) eingebracht, dort mit den Verteilermitteln (15) auf die unterschiedlichen Dekontaminationskammern (3, 4, 5, 6) verteilt und nach einer vorgegebenen Dekontaminationszeit, während derer die Dekontaminationskammern (3, 4, 5, 6) geschlossen sind mit den Transportmitteln (16) innerhalb des Ausschleuseraums (12), bevorzugt in Richtung zu der Ausschleuseöffnung (2), transportiert werden und dass die Dekontaminationsmittelkonzentration in dem Verteilerraum (11) und/oder dem Ausschleuseraum (12) abgesenkt wird.

9. Verfahren nach Anspruch 9,
**dadurch gekennzeichnet,**
**dass** die Dekontaminationsmittelkonzentration in den Dekontaminationskammern (3, 4, 5, 6) auch während des Be- und Entladens der Dekontaminationskammern (3, 4, 5, 6) oberhalb eines Grenzwertes, insbesondere zwischen 300 ppm und 1200 ppm gehalten wird und/oder dass auf ein aktives Absenken der Dekontaminationsmittelkonzentration in den Dekontaminationskammern (3, 4, 5, 6) vor dem Beladen und/oder Entladen der Dekontaminationskammern (3, 4, 5, 6) verzichtet wird.

10. Verfahren nach einem der Ansprüche 8 oder 9, soweit zrückbezogen auf ein Isolatorsystem nach Anspruch 7,
**dadurch gekennzeichnet,**
**dass** entweder in dem Ausschleuseraum (12) aus den dekontaminierten Produkten Verbrauchsgüter entpackt und unmittelbar oder mittelbar über eine Produktschleuse dem Isolator zugeführt werden, oder dass die dekontaminierten Produkte aus dem Ausschleuseraum (12) in eine Produktschleuse (22) transportiert werden, in der aus den dekontaminierten Produkten Verbrauchsgüter entpackt und dann dem Isolator zugeführt werden, oder dass die dekontaminierten Produkte aus dem Ausschleuseraum (12) unmittelbar oder mittelbar über eine Produktschleuse (22) dem Isolator zugeführt und in dem Isolator die Verbrauchsgüter aus den dekontaminierten Produkten entpackt werden.

## Claims

1. A decontamination device (1) for decontaminating products in the form of consumer goods and/or consumer good packaging having consumer goods located therein prior to supplying the consumer goods into an isolator, the decontamination device comprising several decontamination chambers (3, 4, 5, 6) each having a closable inlet opening (9) and a closable outlet opening (10), and each comprising application means (7) for applying a decontamination agent to the decontamination chambers (3, 4, 5, 6), and the decontamination device (1) comprising a distributor chamber (11) disposed upstream of the decontamination chambers (3, 4, 5, 6) and having a supply opening (24) for products to be decontaminated, the distributor chamber (11) comprising distributor means (15) for distributing the products to the different decontamination chambers (3, 4, 5, 6), and the decontamination device (1) comprising a discharge chamber (12) disposed downstream of the decontamination chambers (3, 4, 5, 6) and having a discharge opening (2) and comprising transport means (16) for transporting the decontaminated products out of the decontamination chambers (3, 4, 5, 6), the distributor chamber (11) and/or the discharge chamber (12) being assigned means (17) for reducing a decontamination agent concentration by means of which the decontamination agent is conveyed through a catalytic converter in air circulation mode and/or decontamination agent is displaced by supplying fresh air.

2. The decontamination device according to claim 1,
**characterized in that**
the decontamination chambers (3, 4, 5, 6) are disposed above one another along a vertical and that the distributor means (15) and the transport means (16) each comprise a product lift by means of which the products can be supplied to the individual decontamination chambers (3, 4, 5, 6) disposed above one another or by means of which the products can be transported from the decontamination chambers (3, 4, 5, 6) to the discharge opening (2).

3. The decontamination device according to any one of the preceding claims,
**characterized in that**
the means (17) for reducing the decontamination agent concentration comprise air circulation generator means for generating an air circulation volume flow through catalyst means (20, 21) for degrading the decontamination agent and/or fresh air supply means for rinsing a chamber volume by means of, in particular sterile, fresh air.

4. The decontamination device according to any one of the preceding claims,
**characterized in that**
a product lock (22) having an entrance door (23) is disposed upstream of the distributor chamber (11), said product lock being loadable with products to be decontaminated, and that the products from the product lock (22) can be supplied to the distributor chamber (11) via the supply opening (24) when the entrance door (23) is closed and/or that a product lock by means of which the consumer goods can be supplied to an isolator and/or in which consumer goods can be unpacked from the decontaminated products, which are realized as secondary consumer good packaging, manually or in an automated manner via unpacking means before said products are supplied to the isolator is disposed downstream of the discharge chamber (12).

5. The decontamination device according to any one of the preceding claims,
**characterized in that**
means for transferring products into the decontamination chambers (3, 4, 5, 6) are provided on the distributor means (15) and/or means for removing the decontaminated products from the decontamination chambers (3, 4, 5, 6) are provided on the transport means (16) and/or means for conveying the products from the respective inlet opening (9) to the respective outlet opening (10) are provided in the decontamination chambers (3, 4, 5, 6).

6. The decontamination device according to any one of the preceding claims,
**characterized in that**
lifting means for lifting the products are disposed in the decontamination chambers.

7. An isolator system for pharmaceutical applications comprising an isolator and a decontamination device (1) according to any one of the preceding claims which is connected to said isolator in such a manner that decontaminated products or consumer goods, which are previously unpacked in a product lock, can be supplied to the isolator in a sterile manner.

8. A method for operating a decontamination device (1) according to any one of claims 1 to 6 and/or an isolator system according to claim 7,
**characterized in that**
products to be decontaminated are introduced into the distributor chamber (11) through the supply opening, in particular one after the other, and that said products are then distributed to the different decontamination chambers (3, 4, 5, 6) in said distributor chamber by means of the distributor means (15) and are transported inside the discharge chamber (12), preferably towards the discharge opening (2), by means of the transport means (16) after a predefined decontamination period, the decontamination chambers (3, 4, 5, 6) being closed during said period, and that the decontamination agent concentration in the distributor chamber (11) and/or in the discharge chamber (12) is reduced.

9. The method according to claim 8,
**characterized in that**
the decontamination agent concentration in the decontamination chambers (3, 4, 5, 6) is also kept above a limit value, in particular between 300 ppm and 1200 ppm, during loading and/or unloading of the decontamination chambers (3, 4, 5, 6) and/or that an active reduction of the decontamination agent concentration in the decontamination chambers (3, 4, 5, 6) before loading and/or unloading the decontamination chambers (3, 4, 5, 6) is omitted.

10. The method according to claim 8 or 9, referring back to an isolator system according to claim 7,
**characterized in that**
consumer goods are either unpacked from the decontaminated products in the discharge chamber (12) and are supplied to the isolator directly or indirectly via a product lock, or that the decontaminated products are transported from the discharge chamber (12) into a product lock (22) in which consumer goods are unpacked from the decontaminated products and then supplied to the isolator, or that the decontaminated products are supplied from the discharge chamber (12) to the isolator directly or indirectly via a product lock (22) and that said consumer goods are unpacked from the decontaminated products in the isolator.

## Revendications

1. Dispositif de décontamination (1) pour décontaminer des produits sous forme de produits de consommation et/ou de suremballages de produits de consommation ayant des produits de consommation situés dans ceux-ci avant le transfert desdits produits de consommation dans un isolateur, le dispositif de décontamination (1) comprenant plusieurs chambres de décontamination (3, 4, 5, 6) ayant chacune une ouverture d'entrée (9) qui peut être fermée et une ouverture de sortie (10) qui peut être fermée, chaque chambre de décontamination (3, 4, 5, 6) comprenant des moyens d'application (7) pour appliquer un agent de décontamination (7) aux chambres de décontamination (3, 4, 5, 6), et le dispositif de décontamination (1) comprenant un espace de distribution (11) disposé en amont des chambres de décontamination (3, 4, 5, 6) et ayant une ouverture d'alimentation (24) pour des produits à décontaminer et comprenant des moyens de distribution (15) pour distribuer les produits aux différentes chambres de décontamination (3, 4, 5, 6), et le dispositif de décontamination (1) comprenant un espace d'évacuation (12) disposé en aval des chambres de décontamination (3, 4, 5, 6) et ayant une ouverture d'évacuation (2) et comprenant des moyens de transport (16) pour transporter les produits décontaminés hors des chambres de décontamination (3, 4, 5, 6), des moyens (17) destinés à réduire une concentration d'agent de décontamination étant assignés à l'espace de distribution (11) et/ou à l'espace d'évacuation (12), l'agent de décontamination étant transporté à travers un catalyseur dans un mode de circulation d'air et/ou de l'agent de décontamination étant déplacé par l'alimentation en air frais au moyen des moyens (17) destinés à réduire la concentration d'agent de décontamination.

2. Dispositif de décontamination selon la revendication 1,
**caractérisé en ce que**
les chambres de décontamination (3, 4, 5, 6) sont disposées l'une au-dessus de l'autre le long d'une verticale et que les moyens de distribution (15) et les moyens de transport (16) comprennent chacun un ascenseur de produit au moyen duquel les chambres de décontamination (3, 4, 5, 6) individuelles, qui sont disposées l'une au-dessus de l'autre, peuvent être alimentés en produits ou au moyen duquel les produits peuvent être transportés des chambres de décontamination (3, 4, 5, 6) à l'ouverture d'évacuation (2).

3. Dispositif de décontamination selon l'une quelconque des revendications précédentes,
**caractérisé en ce que**
les moyens (17) destinés à réduire la concentration d'agent de décontamination comprennent des moyens de génération de circulation d'air pour générer un débit de volume de circulation d'air à travers des moyens de catalyseur (20, 21) afin de dégrader l'agent de décontamination et/ou des moyens d'alimentation en air fris pour rincer un volume d'espace au moyen d'air frais, notamment stérile.

4. Dispositif de décontamination selon l'une quelconque des revendications précédentes,
caractérisé en
qu'une écluse de produit comprenant une porte d'entrée (23) est disposée en amont de l'espace de distribution (11), l'écluse de produit (22) pouvant être remplie de produits à décontaminer et les produits pouvant être transférés de l'écluse de produit (22) à l'espace de distribution (11) par l'ouverture d'alimentation (24) quand la porte d'entrée (23) est fermée et/ou qu'une écluse de produit est disposée en aval de l'espace d'évacuation (12), les produits de consommation pouvant être transférés à un isolateur par l'écluse de produit et/ou des produits de consommation pouvant être déballés des produits décontaminés qui sont réalisés comme suremballage de produits de consommation dans l'écluse de produit de manière manuelle ou automatisée par des moyens de déballage avant le transfert à l'isolateur.

5. Dispositif de décontamination selon l'une quelconque des revendications précédentes,
**caractérisé en ce que**
des moyens destinés à transférer des produits dans les chambres de décontamination (3, 4, 5, 6) sont prévus sur les moyens de distribution (15) et/ou que des moyens destinés à enlever des produits décontaminés des chambres de décontamination (3, 4, 5, 6) sont disposés sur les moyens de transport (16) et/ou que des moyens destinés à acheminer les produits de la direction de l'ouverture d'entrée (9) respective à l'ouverture de sortie (10) respective sont prévus dans les chambres de décontamination (3, 4, 5, 6).

6. Dispositif de décontamination selon l'une quelconque des revendications précédentes,
**caractérisé en ce que**
des moyens de levage pour lever les produits sont disposés dans les chambres de décontamination.

7. Système d'isolateur pour des applications pharmaceutiques, le système d'isolateur comprenant un isolateur et un dispositif de décontamination (1) selon l'une quelconque des revendications précédentes relié à l'isolateur de telle manière que des produits décontaminés ou des produits de consommation déballés préalablement des produits dans une écluse de produit peuvent être transférés du dispositif de décontamination (1) à l'isolateur de manière stérile.

8. Procédé de fonctionnement d'un dispositif de décontamination (1) selon l'une quelconque des revendications 1 à 6 et/ou d'un système d'isolateur selon la revendication 7,
**caractérisé en ce que**
des produits à décontaminer sont introduits, notamment l'un après l'autre, dans l'espace de distribution (11) à travers l'ouverture d'alimentation, sont distribués aux différentes chambres de décontamination (3, 4, 5, 6) au moyen des moyens de distribution (15) dans l'espace de distribution (11) et sont transportés au moyen des moyens de transport (16) dans l'espace d'évacuation (12), de préférence vers l'ouverture d'évacuation (2), après un temps de décontamination défini pendant lequel les chambres de décontamination (3, 4, 5, 6) sont fermées et que la concentration d'agent de décontamination dans l'espace de distribution (11) et/ou dans l'espace d'évacuation (12) est réduite.

9. Procédé selon la revendication 8,
**caractérisé en ce que**
la concentration d'agent de décontamination dans les chambres de décontamination (3, 4, 5, 6) est maintenue au-delà d'une valeur limite, notamment entre 300 ppm et 1200 ppm, même pendant le chargement et le déchargement des chambres de décontamination (3, 4, 5, 6) et/ou qu'une réduction active de la concentration d'agent de décontamination dans les chambres de décontamination (3, 4, 5, 6) n'est pas effectuée avant le chargement et/ou le déchargement des chambres de décontamination (3, 4, 5, 6).

10. Procédé selon la revendication 8 ou la revendication 9, en se référant à un système d'isolateur selon la revendication 7,
**caractérisé en ce que**
des produits de consommation sont déballés des produits décontaminés dans l'espace d'évacuation (12) et sont transférés à l'isolateur directement ou indirectement par une écluse de produit, ou que les produits décontaminés sont transportés de l'espace d'évacuation (12) dans une écluse de produit (22) dans laquelle des produits de consommation sont déballés des produits décontaminés et sont ensuite transférés à l'isolateur, ou que les produits décontaminés sont transférés de l'espace de sortie (12) à l'isolateur directement ou indirectement par une écluse de produit (22) et les produits de consommation sont déballés des produits décontaminés dans l'isolateur.
